Europäisches Patentamt

European Patent Office

Office européen des brevets

(11) Publication number: **0 062 962**
**B1**

(12) EUROPEAN PATENT SPECIFICATION

(45) Date of publication of patent specification: **29.01.86**

(51) Int. Cl.⁴: **A 61 B 6/14**

(21) Application number: **82300773.7**

(22) Date of filing: **16.02.82**

(54) **A method to be used in panoramic X-ray photography and an apparatus for carrying out the method.**

(30) Priority: **20.02.81 FI 810530**

(43) Date of publication of application:
**20.10.82 Bulletin 82/42**

(45) Publication of the grant of the patent:
**29.01.86 Bulletin 86/05**

(84) Designated Contracting States:
**AT BE CH DE FR GB IT LI LU NL SE**

(56) References cited:
**EP-A-0 002 706**
**EP-A-0 035 307**
**NL-A-7 904 882**
**US-A-4 039 837**
**US-A-4 145 611**

(73) Proprietor: **Tammisalo, Erkki**
**Linnunpaatie 3**
**SF-20840 Turku 84 (FI)**

(72) Inventor: **Tammisalo, Erkki**
**Linnunpaatie 3**
**SF-20840 Turku 84 (FI)**

(74) Representative: **Cross, Rupert Edward Blount**
**et al**
**BOULT, WADE & TENNANT 27 Furnival Street**
**London EC4A 1PQ (GB)**

## Description

The present invention relates to a method to be used in panoramic X-ray photography by using an X-ray apparatus which performs a rotational movement and comprises an X-ray source and a moving film, by means of which the point of sharpness or focus can be adjusted to a selected position in relation to the patient before photographing is started. The patient is supported by means of support members and, for the purpose of adjustment, a sighting member is moved to the selected point in relation to the patient. The invention also relates to an apparatus for carrying out this method.

The invention relates in particular to panoramic tomography, i.e. photography of a patient's denture, but it can also be applied to other cases of X-ray photography in which an X-ray source and a film, situated on different sides of the patient, are rotated around the patient during the photographing, while the film is reeled forwards.

It is also known from the document US—A—4,145,611 that the form of the layer which is photographed with precision can be affected by means of the geometry of the X-ray apparatus, and especially in panoramic tomography apparatus, by making arrangements for a combined linear and rotational movement of the support arm which supports the X-ray source and the film cassette at its opposite ends.

In order to bring a subject patient and the apparatus to their correct positions in relation to each other before photographing is started, it has been known from the document NL—A—7904882 to use a device to indicate the optical object to be photographed and to move the patient to the correct point according to this device. The disadvantages of this method are the structural requirements set by it on the patient-supporting members and the moving of the patient after initial setting of the apparatus, resulting in the lengthening of the time used for the photographing and sometimes also in an imprecise X-ray photograph.

An alternative to avoid moving the patient is to connect the suspending part of the support arm of the X-ray apparatus mechanically to the sighting device, the patient is always placed in the same position by means of stationary support members and fine adjustment is carried out by moving the geometry of the X-ray apparatus itself. The disadvantage of this alternative is its structurally complicated and expensive moving mechanism, since after initial setting up, the X-ray apparatus, in other words, the support arm, further performs the said combined linear and rotational movement.

An apparatus for carrying out panoramic X-ray photography is also known from the document US—A—4,039,837, in which the velocity of the film is controlled in such a manner that at each given moment it corresponds to the velocity of the projected image of the point being photographed as projected onto the film surface. The film velocity is controlled on the basis of the specific shape of the curve along which the photography is to be taken. This specific shape is selected by comparing with a set of model curves the shape of the object to be photographed, which has to be collected from the patient prior to performing the photography.

The object of the present invention is to eliminate the above-mentioned disadvantages of known apparatus in such a manner that the patient can be placed in a stationary position by means of support members of the apparatus, and that the moving part of the X-ray apparatus, for example, the support arm, can perform the same movement without fine adjustment of the initial point before photographing is started. This objective is achieved by taking advantage of the effect of the film velocity in relation to the optical object being photographed. By adjustments of the film velocity it is possible to shift the location of the optical object to be photographed, e.g. a denture, without adjustment of the patient and the X-ray apparatus geometry.

The main characteristics of the method according to the invention are given in accompanying Claim 1, and the characteristics of the apparatus are respectively given in Claim 3.

The invention and its other characteristics are described below in greater detail in the form of an example and with reference to the accompanying drawings, in which

Figure 1 is a diagrammatic representation of a side elevation of an X-ray apparatus intended for photographing a denture, and;

Figure 2 is a block diagram of the circuitry of the apparatus according to the invention.

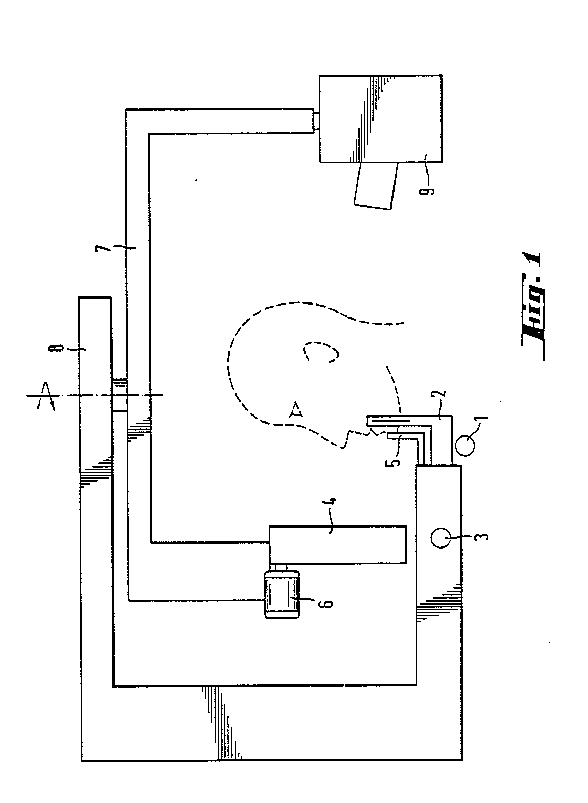

The apparatus depicted in Figure 1 includes the following parts: A position sensor 1, a viewing member 2 for indicating the optical object layer to be photographed, a moving mechanism 3 for the viewing member, an image registration apparatus 4 consisting of a moving film cassette, a patient support 5 for a subject patient which is stationary in relation to the supporting frame, a drive motor 6 for the image registration device, a rotating support arm 7, a supporting frame 8, and an X-ray source 9. The support 5 can, of course, be replaceable depending on what is to be photographed at a given time.

In this X-ray apparatus, the support arm 7 with its X-ray source 9 and film 4 rotates, during the photographing around the subject's head according to a predetermined geometry. Since, as regards this movement, the apparatus is completely known for an expert in the art, reference is made here as regards its operation only by way of example to previous Finnish Patent Application No. 763569, and to the corresponding US Patent US—A 4,145,611.

That which is essential in the present invention is that the subject patient is always placed in a stationary position with the aid of supporting member 5, and that the moving mechanism 3 for the viewing member does not affect the position of the support arm itself but only the control of the drive motor 6, preferably electronically.

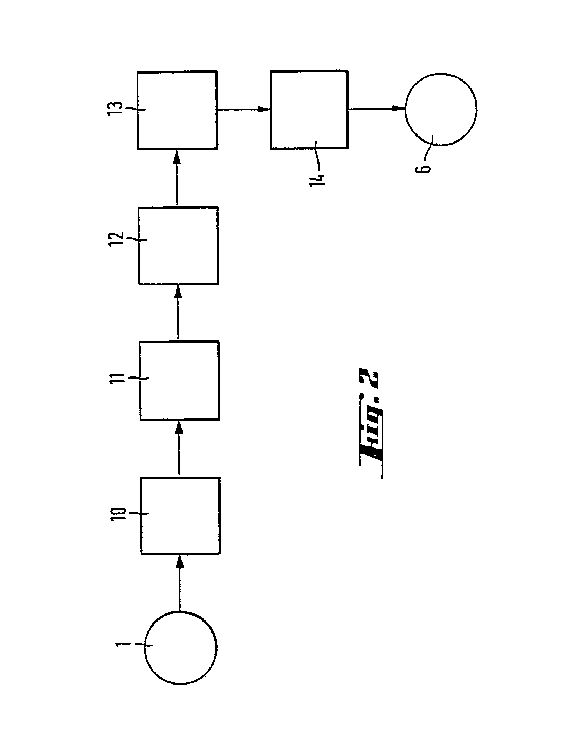

In Figure 2, the block diagram of the circuitry is depicted in greater detail. It thus includes a position sensor 1 connected to the viewing member, a converter 10, which can be an analog/digital converter or, for example, a voltage/frequency converter, a transfer line 11, a computing unit 12, a microprocessor 13, and a motor control unit 14.

The output of the position sensor 1, which indicates the position of the viewing device adjusted to the selected position in relation to the subject, can be, for example, an analog voltage signal, as is well known in the art of control techniques and electronics. This signal is converted in the converter 10 either to a corresponding digital signal or to a frequency signal, the frequency of which corresponds to the position of the indicator. The transfer line 11, which can be a suitable cable, transfers the converter output signal to the computing unit 12, which operates in conjunction with the microprocessor. The control programs for the drive motor 6 have been programmed into the microprocessor, and the programs are selected on the basis of the data obtained from the computing unit 12, i.e. on the basis of the actual position of the optical object to being photographed. The processor can, of course, have fixed circuitry or be freely programmable. The motor 6 can be a step motor into which the motor control unit 14 feeds control pulses at a varying frequency controlled by the microprocessor 13. It is, of course, also possible to use as the drive motor a DC motor controlled by means of a varying voltage.

In currently used X-ray apparatus, mechanical transmission which changes according to the movement of the support arm is generally used for the control of the film velocity. It is evident that such mechanical transmission can also be applied to the present invention in such a manner that the initial setting of the transmission ratio is carried out on the basis of the reading of the position sensor.

**Claims**

1. A method to be used in panoramic X-ray photography for adjusting the layer which is photographed with precision to a selected position in relation to the patient before photographing is started, and of the type wherein an X-ray device (7) performs a rotational movement and comprises an X-ray source (9) and a moving film (4), and wherein the patient is supported by means of support members (5) and, for the purpose of adjustment, a viewing member (2) is moved to indicate the selected position in relation to the patient, characterised in that the support members (5) and the suspending point of the X-ray device (7) are maintained in a stationary position in relation to each other and the preadjustment of the layer which is photographed with precision is performed by adjusting, on the basis of the position or reading of the viewing member (2), the film velocity in such a manner that the film velocity corresponds to the velocity of the image of a point in said selected position as projected on the film surface.

2. A method according to Claim 1, wherein an electric motor (6) which reels the film is controlled by means of a microprocessor (13) to which control signals are fed, said signals being dependent on the position or reading of the viewing member (2).

3. An apparatus for carrying out panoramic X-ray photography, said apparatus comprising;

a stationary frame (8);

an X-ray device (7) provided on the frame (8) and having a suspending part for enabling a combined linear and rotational movement of the X-ray device, said X-ray device comprising;

an X-ray source (9) and a film holder (4) with film-reeling means (6);

patient supporting members (5);

a viewing member (2) for aiming at a selected position of the patient;

characterised by means connected to said viewing member and functioning to adjust the film velocity in response to a signal from said viewing member (2) in such a manner that the film velocity corresponds to the velocity of the image of a point in said selected position as projected on the film surface while both the patient supporting members (5) and the suspending part of the X-ray device (7) remains stationary in relation to the frame (8).

4. An apparatus according to Claim 3, wherein the film-reeling member is an electric motor (6) which is controlled by said means connected to said viewing member (2) which comprises an electronic circuit (10—14), the electronic circuit having a control input to which said signal corresponding to the position or reading of the viewing member (2) is supplied.

5. An apparatus according to Claim 4, wherein the electronic circuit includes a microprocessor (13) in which certain control programs for said electric motor have been programmed, the parameter of the program being derived from the position or reading of the viewing member (2).

**Revendications**

1. Procédé utilisé pour les prises de vue panoramiques, permettant d'ajuster la plage optique qui est photographiée avec précision à une position déterminée par rapport au patient avant de commencer la prise de vue, et du type dans lequel un dispositif à rayons X (7) effectue un mouvement rotatif et comprend une source à rayons X (9) et un film mobile (4), et dans lequel le patient est maintenu en place au moyen d'éléments supports (5), avec dans le but de réaliser l'ajustement, un élément de visée (2) qu'on déplace pour indiquer ladite position déterminée par rapport au patient, caractérisé en ce que les éléments supports (5) et la partie suspendue du dispositif à rayons X (7) sont maintenus dans une position fixe l'un par rapport à l'autre, et le pré-ajustement de la plage optique qui est photographié avec précision est réalisé en ajustant, en fonction de la position ou du relèvement de l'élément de visée (2), la vitesse du film de telle manière que ladite vitesse corresponde à la vitesse de l'image d'un point se trouvant dans ladite position déterminée telle que projeté à la surface du film.

2. Procédé selon la revendication 1 dans lequel un moteur électrique (6) qui enroule le film est commandé au moyen d'un microprocesseur (13) alimenté en signaux de commande, lesdits signaux étant dépendants de la position ou du relèvement de l'élément de visée (2).

3. Appareil pour réaliser des prises de vues panoramiques par rayons X, ledit appareil comprenant:
— un cadre fixe (8);
— un dispositif à rayons X (7) monté sur le cadre (8) et présentant une partie suspendue permettant un mouvement linéaire rotatif combiné du dispositif à rayons X, ledit dispositif à rayons X comprenant:
— une source à rayons X (9) et un support de film (4) avec des moyens (6) d'enroulement du film;
— des éléments de support du patient (5);
— un élément de visée (2) pour obtenir la position déterminée du patient;
caractérisé en ce qu'il comprend des moyens connectés audit élément de visée et fonctionnant de façon à ajuster la vitesse du film en réponse à un signal provenant dudit élément de visée (2), de telle manière que la vitesse du film corresponde à la vitesse de l'image d'un point se trouvant dans ladite position déterminée, telle que projetée sur la surface du film, les éléments de support du patient (5) et la partie suspendue du dispositif à rayons X (7) demeurant fixes par rapport au cadre (8).

4. Appareil selon la revendication 3 dans lequel l'élément d'enroulement du film est un moteur électrique (6) qui est commandé par lesdits moyens connectés audit élément de visée (2), lequel comprend un circuit électronique (10—14), ledit circuit électronique ayant une entrée de commande recevant ledit signal correspondant à la position ou au relèvement de l'élément de visée (2).

5. Appareil selon la revendication 4, lequel le circuit électronique comprend un microprocesseur (13) dans lequel on a programmé certains programmes de commande dudit moteur électrique, les paramètres du programme découlant de la position ou du relèvement de l'élément de visée (2).

**Patentansprüche**

1. Verfahren für die Panorama-Röntgenfotografie zum Einstellen der scharf abzubildenden Schicht auf eine ausgewählte Stelle in Bezug auf einen Patienten vor Beginn des Aufnahmevorgangs, bei welchem Verfahren die Röntgeneinrichtung (7) eine Drehbewegung ausführt und eine Röntgenquelle (9) und einen beweglichen Film (4) aufweist, wobei der Patient durch Stützelemente (5) gestützt wird und zum Zweck der Einstellung ein Sichtelement (2) zur Anzeige der ausgewählten Stelle in Bezug auf den Patienten bewegt wird, dadurch gekennzeichnet, daß die Stützelemente (5) und der Aufhängungspunkt für die Röntgeneinrichtung (7) in einer festen Position relativ zueinander gehalten werden und die Vor-Einstellung der scharf fotografierten Schicht ausgeführt wird, indem auf der Grundlage der Position oder Anzeige des Sichtelements (2) die Filmgeschwindigkeit derart eingestellt wird, daß die Filmgeschwindigkeit der Geschwindigkeit des auf die Filmoberfläche projizierten Bildes eines Punktes an der genannten ausgewählten Stelle entspricht.

2. Verfahren nach Anspruch 1, bei dem ein Elektromotor (7), der den Film transportiert, mit Hilfe eines Mikroprozessors (13) gesteuert wird, dem Steuersignale zugeführt werden, welche Signale von der Position oder Anzeige des Sichtelements (2) abhängen.

3. Gerät für Panorama-Röntgen-Aufnahmen, das ein feststehendes Gestell (8),
eine Röntgeneinrichtung (7), die auf dem Gestell (8) angeordnet ist und ein Aufhängungsteil besitzt, das eine kombinierte lineare Bewegung und Drehbewegung der Röntgeneinrichtung gestattet, welche Röntgeneinrichtung
eine Röntgenquelle (9) und einen Filmhalter (4) mit einer Film-Transporteinrichtung (6) umfaßt,
Patienten-Stützelemente (5),
ein Sichtelement (2) zum Zielen auf eine ausgewählte Stelle des Patienten aufweist,
gekennzeichnet durch eine mit dem Sichtelement verbundene Einrichtung, die die Filmgeschwindigkeit entsprechend einem Signal von dem Sichtelement (2) derart einstellt, daß die Filmgeschwindigkeit der Geschwindigkeit des auf die Filmoberfläche projizierten Bildes eines Punktes an der genannten ausgewählten Stelle entspricht, während sowohl die Patienten-Stützelemente (5) als auch das Aufhängungsteil der Röntgeneinrichtung (7) stationär in Bezug auf das Gestell (8) gehalten werden.

4. Gerät nach Anspruch 3, bei dem die Film-Transporteinrichtung ein Elektromotor (6) ist, der durch die genannte, mit dem Sichtelement (2) verbundene Einrichtung gesteuert wird, die eine elektronische Schaltung (10—14) umfaßt, welche elektronische Schaltung einen Steuereingang aufweist, dem das genannte, der Position oder Anzeige des Sichtelements (2) entsprechende Signal zugeführt wird.

5. Gerät nach Anspruch 4, bei dem die elektronische Schaltung einen Mikroprozessor (13) aufweist, in den bestimmte Steuerprogramme für den genannten Elektromotor einprogrammiert worden sind, wobei der Parameter des Programms von der Position oder Anzeige des Sichtelements (2) abgeleitet ist.

Fig. 1

0 062 962

Fig. 2